# EUROPEAN PATENT SPECIFICATION

(11) **EP 3 773 270 B1**
(45) Date of publication and mention of the grant of the patent: **12.02.2025**
(21) Application number: 19771954.5
(22) Date of filing: 19.03.2019
(51) Int. Cl.: A61B 17/3207, A61B 17/3209, A61F 2/04, A61B 17/221, A61B 17/22, A61B 17/00, A61B 90/00

(54) **URETHRAL IMPLANT DELIVERY SYSTEM**
SYSTEM ZUR EINFÜHRUNG EINES HARNRÖHRENIMPLANTATS
SYSTÈME POUR LA POSE D'IMPLANT URÉTRAL

(30) Priority: 19.03.2018 US 201862645024 P; 18.03.2019 US 201962819712 P
(43) Date of publication of application: 17.02.2021
(73) Proprietor: Medi-Tate Ltd., 3850169 Hadera (IL)
(72) Inventor: KILEMNIK, Ido, 4286500 Haniel (IL)
(74) Representative: Hoener, Matthias
(86) International application number: PCT/IL2019/050306
(87) International publication number: WO 2019/180711

(56) References cited:
- US-A1- 2015 127 050
- US-A1- 2015 257 908
- US-A1- 2016 317 180
- US-A1- 2017 000 598
- US-A1- 2017 105 607
- US-B2- 6 491 672

## Description

### FIELD OF THE DISCLOSED TECHNIQUE

The disclosed technique relates to system and method for treating a prostate enlargement (e.g., as a result of benign prostatic hyperplasia), in general, and to systems and methods for creating incisions in the muscles of the bladder neck and the prostatic urethra, in particular.

### BACKGROUND OF THE DISCLOSED TECHNIQUE

The prostate is a walnut-sized gland that forms part of the male reproductive system. The prostate is located in front of the rectum and just below the bladder, where urine is stored. The prostate surrounds the urethra, the canal through which urine passes out of the body. Prostate enlargement can result from a number of medical problems such as benign prostatic hyperplasia (BPH), prostatic Bladder neck obstruction (BNO) and the like. The enlarged prostate applies pressure on the urethra and damages bladder function.

Transurethral incision of the prostate (TUIP) is an endoscopic procedure usually performed under general anaesthetic in which a surgeon employs an instrument (e.g., a scalpel, a laser beam generator and an electrical current actuator) inserted into the urethra for making incisions in the bladder neck where the prostate meets the bladder (i.e., more specifically in the midline to the level of the verumontanum). Incising the muscles in the bladder neck area relieves the obstructive effect of the prostate on the bladder neck and prostatic urethra and relaxes the opening of the bladder, thus decreasing resistance to the flow of urine out of the bladder. It is noted that, no tissue is removed during TUIP.

Infarction is a process resulting in a macroscopic area of necrotic tissue in some organ caused by loss of adequate blood supply. The inadequate blood supply can result from pressure applied to the blood vessels. Even by applying a relative small but continuous pressure on a tissue, one can block the tiny blood vessels within the tissue and induce infarction.

PCT International Patent Application Publication No. WO 2006/040767 A1 co-invented by the inventor of the present patent application, entitled "Prostate Treatment Stent" is directed at a tissue dissecting implant kit. The tissue dissecting implant kit includes an implant and a sterile package. The implant includes a plurality of rings elastically coupled there-between. An elastic pressure is applied on tissue caught between adjacent rings. The sterile package encompasses the implant. The implant has different distances between adjacent rings along its length. Alternatively, the implant has different material thickness or cross-section shape along its length. It is noted that, the tissue dissecting implant kit applies pressure on tissue caught between adjacent rings until the tissue is cut away or until the tissue falls off.

US Patent Application Publication No. 2011/0276081 by the inventor of the present patent application, entitled "Radial Cutter Implant" is directed at an implant for applying radial forces on the tissues surrounding it. The implant includes wire strings coupled between a proximal end and a distal end. The wires apply radial pressure on the surrounding tissues. In particular, each wire extends from the proximal end of the implant to the distal end of the implant. The implant can further include a longitudinal tube for providing structural stability to the implant wires. In some embodiments, each wire is in the shape of a butterfly wing, thereby fixing the implant in place within the bladder neck. Note that in those embodiments a portion of the wire sits within the bladder itself for preventing the implant from sliding in the proximal direction. Thereby, the portions of the wires coming into touch with the tissues of the bladder may irritate the bladder.

US Patent No. 5,209,725 issued to Roth, and entitled "Prostatic Urethra Dilatation Catheter System and Method" is directed to an instrument for performing a transurethral balloon dilatation procedure of the prostate. The balloon dilatation instrument includes a hollow catheter and optical viewing means. The hollow catheter includes a shaft, an inflatable optically transparent balloon, and at least one suitable visible marking.

The distal end portion of the shaft is made of an optically transparent material. The inflatable optically transparent balloon is coupled with the distal end portion of the shaft, and is sized to dilate the prostatic urethra. The at least one suitable visible marking is positioned on the catheter proximally to the balloon, such that the marking can be visualized relative to a predetermined anatomical landmark (e.g., verumon-tanum). In this manner, proper positioning of the balloon, relative to the prostatic urethra, is performed prior to and during the dilation of the prostatic urethra. The optical viewing means, is slidable within the catheter, for visibly viewing the marking intra-luminally from within the catheter. The balloon is correctly located relative to the prostatic urethra. The balloon is inflated so as to dilate the prostatic urethra without damaging the external sphincter at the apex of the prostate.

US Patent No. 5,499,994 issued to Tihon et al., and entitled "Dilation Device for the Urethra" is directed to a dilation device for opening a portion of an obstructed urethra. The dilation device includes an inner hollow tubular core and an outer confining covering. The inner hollow tubular core defines a lumen therein. The lumen is a conduit of sufficient covering is capable of expanding radially outwardly to a predetermined extent. The covering has a length of at least partially that of the obstructed portion of the urethra. The dilation device can further include retractable spikes for anchoring the device in its intended position.

U.S. Patent Application Publication No.: US 2016/0317180 A1, titled "An Incising Implant for the Prostatic Urethra" to Kilemnik, is directed at an incising implant and method for creating incisions in the prostatic urethra of a subject using the incising implant. The implant includes at least two closed-shaped wires that are elastic thereby being compressible into a compressed configuration. The incising implant is configured for implantation within the prostatic urethra and is further configured to apply a radial outward force on the surrounding tissues of the inner walls of the urethra for the creation of longitudinal incisions.

U.S. Patent Application Publication No.: US 2017/0000598 A1, titled "A Dilating Device for Prostatic Urethra" is directed at a dilating device for the prostatic urethra that includes three laterally connected ridges. Each ridge is configured to longitudinally engage with a different longitudinal groove of the prostatic urethra of a patient. The three laterally connected ridges are configured to laterally compress to enable insertion into the prostatic urethra in a compressed configuration. The three laterally connected ridges are configured to laterally expand to a normally-open configuration upon deployment within the prostatic urethra, so to exert a radially outwards force that dilates the prostatic urethra.

### SUMMARY OF THE PRESENT DISCLOSED TECHNIQUE

It is an object of the disclosed technique to provide a method and system for delivering a urethral implant. In accordance with the disclosed technique, there is thus provided a urethral implant delivery system including an overtube, a camera, a urethral implant and a guidewire. The overtube includes a first elongated passage, a second elongated passage and a handle connector. The camera is located at the distal end of the first elongated passage. The urethral implant is located within the second elongated passage. The guidewire is coupled with the urethral implant and extends toward the proximal end of the overtube. The handle connector is configured to be coupled with a handle.

The urethral implant delivery system according to the invention is defined in claim 1. Embodiments are defined in the dependent claims. The methods disclosed are not recited by the wording of the claims but are useful for understanding the invention.

### BRIEF DESCRIPTION OF THE DRAWINGS

The disclosed technique will be understood and appreciated more fully from the following detailed description taken in conjunction with the drawings in which Figures 10A-10C disclose a system according to the invention:
Figure 1 is a schematic illustration of an overtube for determining the location of a bladder neck of a patient and delivering a radial cutter implant thereto, constructed and operative in accordance with an embodiment of the disclosed technique;
Figure 2 is a schematic illustration of a delivery for delivering a radial cutter implant to the bladder neck of a patient, constructed and operative in accordance with another embodiment of the disclosed technique;
Figures 3A, 3B and 3C are schematic illustrations of a system for delivering a radial cutter implant to the bladder neck of a patient, constructed and operative in accordance with a further embodiment of the disclosed technique;
Figures 4A, 4B and 4C are schematic illustrations of a delivery for delivering a radial cutter implant, constructed and operative in accordance with another embodiment of the disclosed technique;
Figures 5A, 5B and 5C are schematic illustrations of a radial cutter implant, constructed and operative in accordance with a further embodiment of the disclosed technique;
Figure 6 is a schematic illustration of a method for creating incisions in the muscles of the bladder neck by infarction, operative in accordance with a further embodiment of the disclosed technique;
Figure 7 is a schematic illustration of a radial cutter implant, depicted from a top view perspective, constructed and operative in accordance with a further embodiment of the disclosed technique;
Figures 8A and 8B are schematic illustrations of a radial cutter implant, constructed and operative in accordance with another embodiment of the disclosed technique;
Figures 9A-9L are schematic illustrations of a step-by-step method for delivering, and for extracting, a radial cutter implant, generally referenced 420, operative in accordance with a further embodiment of the disclosed technique;
Figures 10A, 10B and 10C, are schematic illustrations of a system for delivering a urethral implant to the bladder neck of a patient, constructed and operative in accordance with another embodiment of the disclosed technique;
Figures 11A-11E, are a schematic illustrations of an overtube according to a further embodiment of the disclosed technique; and
Figures 12A-12D, are schematic illustrations of different cross section configurations of an overtube, in accordance with another embodiment of the disclosed technique.

### DETAILED DESCRIPTION OF THE EMBODIMENTS

The disclosed technique overcomes the disadvantages of the prior art by providing an implant for applying small yet continuous pressure on the tissues of the bladder neck sphincter (i.e., as well as tissues of the urethra and the prostate gland) by a plurality of wires. The pressure induces infarction in the tissues (i.e., tissues of the bladder neck, urethra, and prostate gland) which creates a plurality of desired incisions (i.e., each of the wires creates an incision). The incisions relive a prostate enlargement problem by cutting through the tissues and extending the urinal passage (i.e., the wires both incise and extend the tissues in the radial direction from the urethra axis outwardly). The disclosed technique further includes a delivery and deployment system for the incising implant. It is noted that, in this application, a radial cutter implant which applies pressure on the tissues of the bladder neck, further applies pressure on the tissues of the prostate and urethra unless specifically mentioned otherwise along the text.

The terms pressure and force (e.g., applying radial pressure and applying radial force) are employed interchangeably herein below, to describe the operation of the wires of the implant on the surrounding tissues, and vice versa. That is, the wires are described as applying pressure on the tissues, or as applying force on the tissues. Herein below, the terms proximal and distal refer to directions relative to implantable device and the delivery system. In particular, the distal end is the end of the device (or of the system) that is inserted into the body of the patient first and reaches the deepest. The proximal end is the end closer to the exit from the body of the patient.

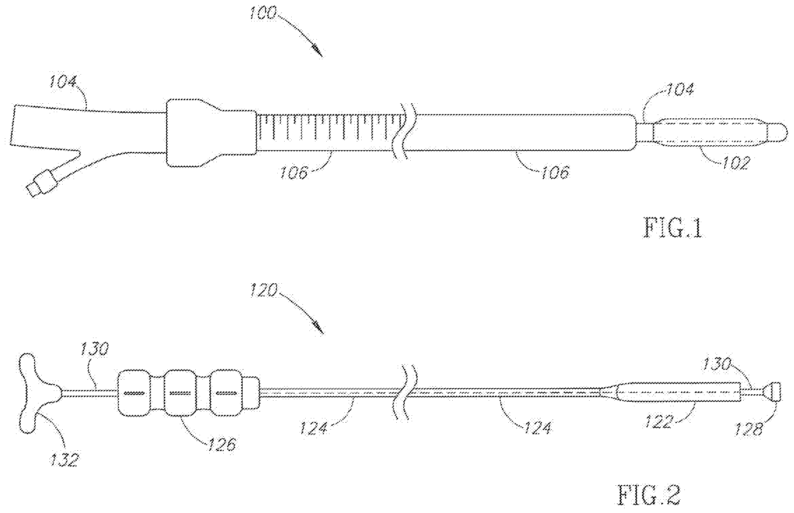

Reference is now made to Figure 1, which is a schematic illustration of an overtube, generally referenced 100, for determining the location of a bladder neck of a patient and delivering a radial cutter implant thereto, constructed and operative in accordance with an embodiment of the disclosed technique. Overtube 100 includes a balloon 102, a balloon tube 104 (i.e., balloon Foley-catheter 104), and a positioning tube 106. Balloon 102 is coupled around balloon tube 104. Balloon tube 104 slidably goes through positioning tube 106.

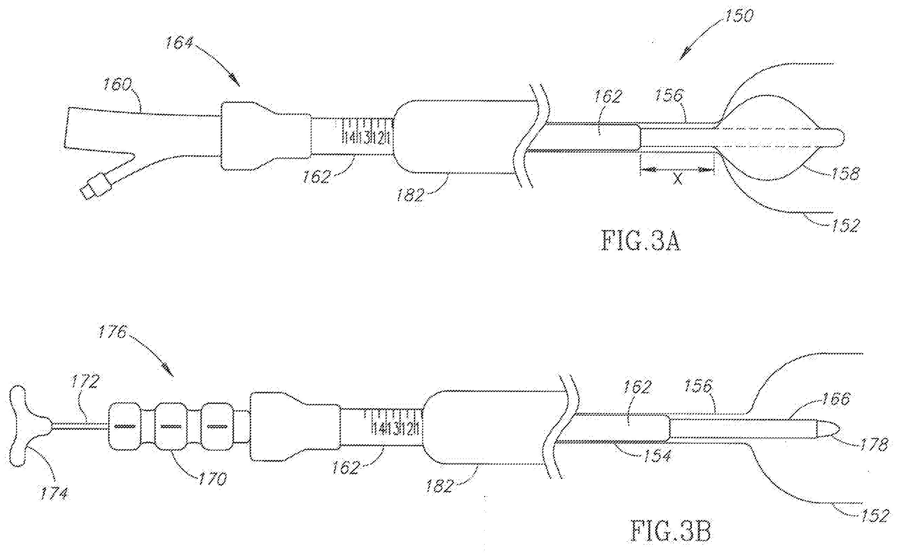

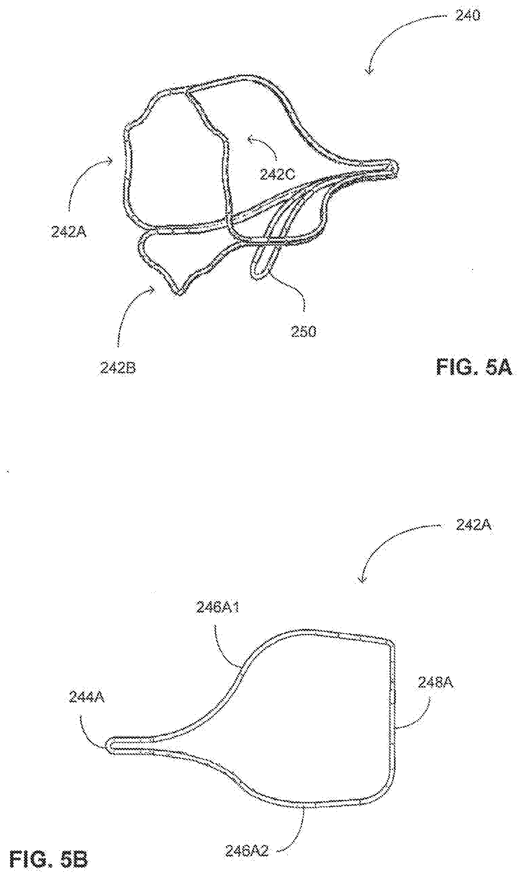

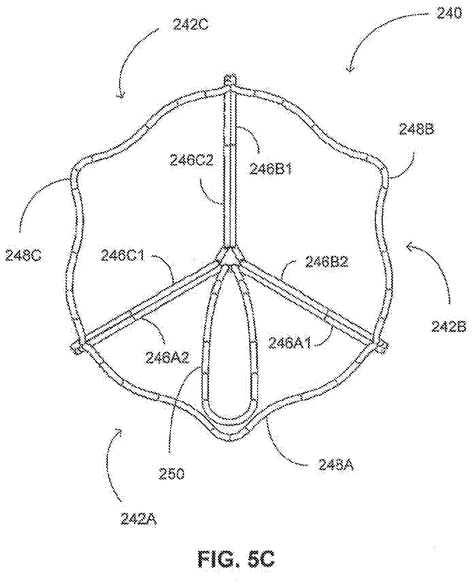

Overtube 100 enables a physician (not shown) to deploy a radial cutter implant (e.g., radial cutter implant 240 of Figures 5A, 5B and 5C) at the bladder neck of a patient (both bladder neck and patient are not shown). The physician inserts overtube 100 through the urethra of the patient until balloon 102 is positioned within the bladder (e.g., bladder 152 of Figure 3A) of the patient. The physician inflates balloon 102 via balloon tube 104. When balloon 102 is inflated, the physician pulls overtube 100 in the proximal direction (i.e., the physician pulls overtube 100 back towards him) until inflated balloon 102 is blocked by the bladder neck. Thus, the physician determines the exact position of the bladder neck of the patient. The physician deflates balloon 102 and removes balloon tube 104 from overtube 100 while leaving positioning tube 106 in place. Alternatively, the physician can determine the location of the bladder neck, and position positioning tube 106 accordingly, by employing any method known in the art, such as ureteroscopy, cystoscopy, ultra-sound imaging, fluoroscopy, and the like.

Reference is now made to Figure 2, which is a schematic illustration of a delivery system, generally referenced 120, for delivering a radial cutter implant to the bladder neck of a patient, constructed and operative in accordance with another embodiment of the disclosed technique. Delivery system 120 includes an implant sheath 122, an external tube 124, an external tube handle 126, an internal tube distal end 128, an internal tube 130, and an internal tube handle 132. Implant sheath 122 is coupled with the distal end of external tube 124. External tube handle 126 is coupled with the proximal end of external tube 124. Internal tube distal end 128 is coupled with the distal end of internal tube 130. Internal tube 130 slidably goes through external tube 124. Internal tube handle 132 is coupled with the proximal end of internal tube 130.

A radial cutter implant (not shown - e.g., radial cutter implant 240 of Figures 5A, 5B and 5C) is detachably coupled with internal tube distal end 128 such that the implant is covered by implant sheath 122. In particular, and relating to the configuration of delivery system 120, as depicted in Figure 2, internal tube 130 slides along external tube 124 in the distal direction until implant sheath 122 is positioned adjacent internal tube distal end 128. In this manner implant sheath 122 covers the radial cutter implant, thereby restraining it.

The physician inserts delivery system 120 into the urethra of the patient through positioning tube 106 of Figure 1. The physician employs positioning tube 106 (Figure 1) for positioning the radial cutter implant at the location of the bladder neck (i.e., or of the restricted location of the urethra) as located by employing overtube 100. Once the radial cutter implant is positioned within the bladder neck, the physician exposes the radial cutter implant, as detailed further below with reference to Figures 3A-3C.

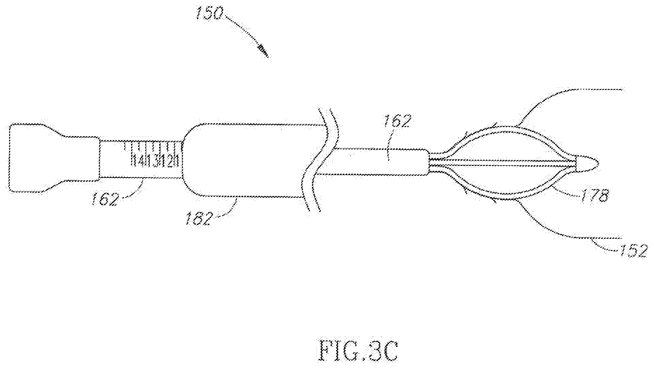

Reference is now made to Figures 3A, 3B and 3C, which are schematic illustrations of a system, generally referenced 150, for delivering a radial cutter implant to the bladder neck of a patient, constructed and operative in accordance with a further embodiment of the disclosed technique. Prior to delivery of the implant, the subject may receive medication to relieve anxiety. The subject may also receive prophylactic antibiotics per local hospital practice. With reference to Figure 3A, delivery system 150 includes an overtube 164, substantially similar to overtube 100 of Figure 1. Overtube 164 includes a balloon 158, a balloon tube 160 and a positioning tube 162. Each of balloon 158, balloon tube 160 and positioning tube 162 is substantially similar to balloon 102, balloon tube 104 and positioning tube 106 of Figure 1, respectively.

The physician inserts overtube 164 into a penis 182 of the patient and through a urethra 154 (Figure 3B) of the patient, until balloon 158 is positioned within a bladder 152 of the patient. The physician inflates balloon 158 via balloon tube 160. Once balloon 158 is inflated, the physician pulls back overtube 164 (i.e., in the proximal direction) until balloon 158 is blocked by bladder neck 156 of the patient. The physician deflates balloon 158 and removes balloon tube 160 from within overtube 164 while keeping positioning tube 162 in place. Thus, the physician locates the exact position of bladder neck 156.

With reference to Figure 3B, delivery system 150 further includes a delivery 176, substantially similar to delivery system 120 of Figure 2. Delivery 176 includes an implant sheath 166, an external tube 168 (located within positioning tube 162 and is not shown in the figure), an external tube handle 170, an internal tube 172, and an internal tube handle 174. Delivery system 150 further includes a radial cutter implant 178 within implant sheath 166. Radial cutter implant 178 may be temporary in the sense that it may be removed from the urethral at a selected time after deployment. Each of implant sheath 166, external tube 168, external tube handle 170, internal tube 172, and internal tube handle 174, is substantially similar to each of implant sheath 122, external tube 124, external tube handle 126, internal tube 130, and internal tube handle 132, respectively.

After removing balloon tube 160 from overtube 164 (Figure 3A), the physician inserts delivery 176 into positioning tube 162. The physician positions delivery 176 such that radial cutter implant 178 is positioned according to the position of positioning tube 162. The physician pulls external tube handle 170 for exposing radial cutter implant 178. Radial cutter implant 178 expands until it is attached to the walls of bladder neck 156 (i.e., to the muscles of bladder neck 156 and the surrounding tissues). Radial cutter implant 178 starts applying pressure to the walls of bladder neck 156 and urethra 154 (i.e., as well as on tissues of the prostate - not shown - as detailed herein above). In the example set forth in Figure 3B, radial cutter implant 178 is self-expanding. Alternatively, radial cutter implant 178 is expanded manually by the physician by employing an expander (i.e., a device for expanding implant 178 as known in the art - for example, a balloon).

With reference to Figure 3C, radial cutter implant 178 is positioned within urethra 154 in an expanded configuration. The physician pulls positioning tube 162 out of the patient and leaves radial cutter implant within urethra 154 for a predetermined period of time (as detailed herein below - e.g., two weeks). Radial cutter implant 178 applies pressure on the walls of the surrounding tissues (e.g., bladder neck 156, urethra 154, and the prostate gland - not shown) incising the surrounding tissues over the predetermined period of time. The prolonged incision of the tissue, created by continuous pressure, decreases the pain involved in the procedure. Furthermore, by performing the incisions via continuous pressure (i.e., via infarction), bleeding is avoided.

The period of time, radial cutter implant 178 is implanted in the urethra of the patient, is determined by the physician at least according to the diagnosis of the patient (i.e., predetermined period of time). Alternatively, the time period is determined according to observations of the radial cutter implant effect over time (i.e., real time period determination), or any other way known in the art. Further alternatively, the time period ranges between one hour and twenty nine days.

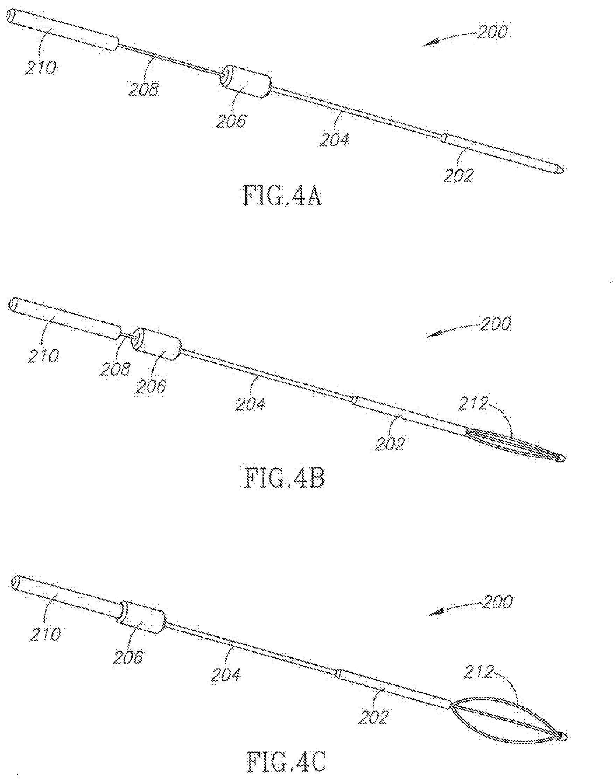

Reference is now made to Figures 4A, 4B and 4C, which are schematic illustrations of a delivery, generally referenced 200, for delivering a radial cutter implant, constructed and operative in accordance with another embodiment of the disclosed technique. With reference to Figure 4A, delivery 200 is substantially similar to delivery system 120 of Figure 2. Delivery 200 includes an implant sheath 202, an external tube 204, an external tube handle 206, an internal tube 208, and an internal tube handle 210. Each of implant sheath 202, external tube 204, external tube handle 206, internal tube 208, and internal tube handle 210 is substantially similar to each of implant sheath 122, external tube 124, external tube handle 126, internal tube 130, and internal tube handle 132 of Figure 2, respectively.

Implant sheath 202 is coupled with the distal end of external tube 204. External tube handle 206 is coupled with the proximal end of external tube 204. A radial cutter implant 212 (Figure 4B) is coupled, at a folded configuration thereof, with the distal end of internal tube 208 and is covered by implant sheath 202. Radial cutter implant 212 may be temporary in the sense that it may be removed from the urethral at a selected time after deployment. Internal tube 208 is slidably coupled with external tube 204. Internal tube handle 210 is coupled with the proximal end of internal tube 208.

With reference to Figure 4B, a physician (not shown) pulls external tube 204 via external tube handle 206 while keeping internal tube 208 in place. Thus, external tube 204 slides along internal tube 208 in the proximal direction and implant sheath 202 is removed from radial cutter implant 212.

With reference to Figure 4C, once implant sheath 202 is fully removed from radial cutter implant 212 (i.e., radial cutter implant is fully exposed), radial cutter implant 212 expands. In the example set forth in Figure 4C, radial cutter implant 212 is self-expanding. Alternatively, radial cutter implant 212 is expanded manually by the physician employing an implant expander (not shown).

The physician leaves radial cutter implant 212 within the body of the patient for a predetermined period of time. When the physician wishes to remove radial cutter implant 212, the physician inserts delivery 200 into the urethra (not shown) of the patient. The physician couples the distal end of internal tube 208 with radial cutter implant 212 by employing a coupler (not shown). The coupler can be any mechanical element that can grab the proximal end of the implant and enable the physician to pull the implant into implant sheath 202. Alternatively, the coupler can be formed of coupling elements that enable the physician to grab the implant and pull it proximally, and that are not mechanical, such as a magnetic coupler.

The physician pulls back internal tube 208 while keeping external tube 204 in place. Thus, radial cutter implant 212 is folded within, and is restrained by, implant sheath 202 and can be extracted from the body (i.e., the bladder neck and the urethra) of the patient, without damaging the tissues of the urethra. It is noted that, the delivery of radial cutter implant 212 and the extraction thereof are substantially a reverse duplicates of each other. In other words, the steps performed upon delivery are repeated in a reverse order upon extraction.

Reference is now made to Figures 5A, 5B and 5C, which are schematic illustrations of a radial cutter implant, generally referenced 240, constructed and operative in accordance with a further embodiment of the disclosed technique. Figure 5A depicts implant 240 from an isometric perspective. Figure 5B depicts a closed shape wire of the implant of Figure 5A from a side view perspective, and Figure 5C depicts implant 240 from a top view perspective.

Implant 240 is constructed from three closed shaped wires 242A, 242B and 242C (also referred to herein below, together, as wires 242), and further includes an anchoring leaflet 250. Each of wires 242 is adjacently attached between the other ones of wires 242, as would be detailed further below. Anchoring leaflet 250 extends from a proximal end (not referenced) of implant 240, and is positioned between two of wires 242. Implant 240 may be temporary in the sense that it may be removed from the urethral at a selected time after deployment.

Each of wires 242A, 242B and 242C includes three sections: a narrow U-shaped proximal end; two radially extending arms - extending radially and distally from the distal ends (not referenced) of the U-shaped proximal end; and a support crosspiece connecting the distal ends of the radially extending arms. In particular and as seen in Figure 5B, closed shape wire 242A includes a U-shaped proximal end 244A, two radially extending arms 246A1 and 246A2, and a support crosspiece 248A.

Radially extending arms 246A1 and 246A2 extend radially and distally from the distal ends (not referenced) of U-shaped proximal end 244A. Support crosspiece 248A is coupled between the distal ends (not referenced) of radially extending arms 246A1 and 246A2. The components of wires 242B and 242C are similarly constructed and denoted.

In this manner and as seen in Figure 5C, support crosspieces 248A, 248B, and 248C form together a support frame (not referenced) on a plane which normal is the longitudinal axis of implant 240 (i.e., the plane depicted in Figure 5C). The support frame of the support crosspieces provides structural solidity to implant 240. Specifically, the support frame provides resistance to forces applied on implant 240 by the surrounding tissues of the bladder neck, when implanted within the bladder neck. In other words, the radial cutter implant, and in particular, the radially extending arms thereof, apply radially outward force on the surrounding tissues for producing incisions in the tissues. Thereby, the surrounding tissues of the bladder neck apply an opposite force on the radially extending arms of the implant. The support frame formed by the support crosspieces maintains the structural stability of the implant, and enables the radially extending arms to continue to apply the incising force on the surrounding tissues.

Support crosspieces 248A, 248B and 248C form together the support frame of supporters. The radially inward force applied by the tissues surrounding implant 240 causes support crosspiece 248A to push against supporters 248B and 248C; support crosspiece 248B to push against supporters 248A and 248C; and causes support crosspiece 248C to push against supporters 248A and 248B. Thereby, supporters 248A, 248B and 248C support each other and resist to radially inward forces applied on the radially extending arms by the surrounding tissues.

During insertion of implant 240 into the bladder neck (and extraction out of), implant 240 is folded with an implant sheath (e.g., implant sheath 202 of Figure 4A). When exposed from the implant sheath, implant 240 expands (or is being expanded) to an expanded configuration depicted in Figures 5A, 5B and 5C. Therefore, implant 240 should be flexible.

Each of closed shape wires 242A, 242B and 242C is made from a shape-memory Alloy (SMA), such as Nickel Titanium alloy (Nitinol). Alternatively, each of wires 242A, 242B and 242C is made from any material which is flexible enough to be folded within an implant sheath and is strong enough (e.g., 0.5 Newton) to apply pressure on the surrounding tissues and induce infarction.

Each of wires 242A, 242B and 242C is flexible such that it can be straightened in order for radial cutter 240 to be folded within an implant sheath (not shown - e.g., implant sheath 202 of Figure 4A). Each of wires 242A, 242B and 242C springs back to expanded state, once not limited by an obstacle (e.g., implant sheath 202 of Figure 4A, the walls of the bladder neck of the patient). In this manner, when radial cutter implant 240 is positioned within the bladder neck of the patient, wires 242A, 242B and 242C, apply pressure to the surrounding tissues (e.g., bladder neck, urethra and prostate).

Alternatively, implant 240 is made of biodegradable materials, such that there is no need to remove implant 240 from the body of the patient. In this manner, implant 240 is constructed such it biodegrades, ceases from functioning and dissolves within the patient after the predetermined period of time, or after a triggering event initiated by the physician, as known in the art.

Anchoring leaflet 250 is in the shape of a tongue extending substantially in the distal-normal direction (i.e., the normal direction refers to a direction normal to the distal-proximal axis - e.g., the dorsal direction). In this manner, anchoring leaflet 250 prevents implant 240 from moving from the bladder neck into the bladder. In particular, anchoring leaflet 250 is blocked by the bladder neck such that implant 240 cannot move into the bladder. It is noted that, anchoring leaflet 250 can be a wire leaflet (e.g., as depicted in Figures 5A to 5C) or a full surface leaflet.

Alternatively, anchoring leaflet 250, extends in the proximal-normal direction and prevents radial cutter implant 240 from moving in the proximal direction towards the urethra. Further alternatively, there are at least two leaflets 250 extending in both directions and fixing implant 240 in place. Anchoring leaflet 250 is constructed of similar materials to those of wires 242.

As mentioned above U-shaped proximal ends 244A, 244B and 244C are coupled together, such that each one is adjacently attached between the other two. In this manner U-shaped proximal ends 244A, 244B and 244C also provide structural solidity to implant 240, similarly to support crosspieces 248A, 248B and 248C. Additionally the U-shape proximal ends 244A, 244B and 244C enable the physician to hook implant 240 by employing a coupler device for extracting implant 240 out of the body of the patient.

As mentioned above, closed shape wires 242A, 242B and 242C are coupled to each other. The wires can be coupled by employing various techniques. For, example the wires can be welded together. The wires can be wound around each other at selected sections thereof (e.g., radially extending arm 246A1 is wound around arm 246B2; arm 246B1 is wound around arm 246C2; and arm 246C1 is wound around arm 246A2). The wires can be glued together. The wires can be coupled by any other coupling mechanism, element or method.

In accordance with an alternative embodiment of the disclosed technique, implant can comprise other numbers of closed shaped wires, and at least one closed shape wire, such as four wires, five wires, and the like. If there is more than a single wire, each wire is adjacently attached between two adjacent wires as described above. Similarly, for any number of closed shape wires, the support crosspieces thereof form together a support frame providing structural solidity to implant, as described above.

Radial cutter implant 240 is located and expanded such that it does not extend distally beyond the bladder neck of the patient (i.e., does not extend into the bladder). Specifically, the support frame formed of the support crosspieces is located within the bladder neck before the bladder, and does not come into contact with the tissues of the bladder itself. Thereby, implant 240 does not irritate the bladder of the patient. It is noted that irritation of the bladder tissues may stimulate urinary activity.

In accordance with another embodiment of the disclosed technique, the radial cutter implant is colored in such a manner that enables the physician to easily position it in order. For example, the wires of the implant are color coded such that sections that should be positioned on top are colored blue, and sections that should be positioned on the bottom are colored white.

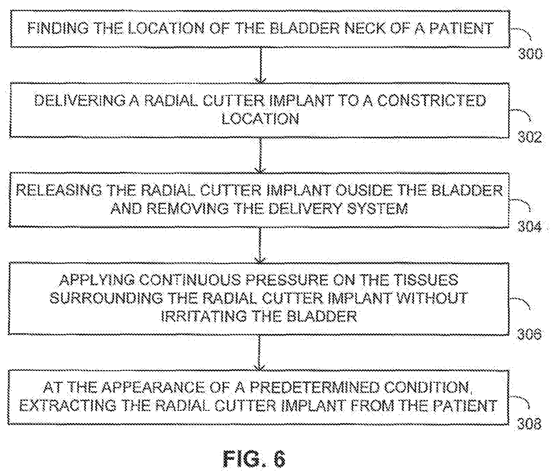

Reference is now made to Figure 6, which is a schematic illustration of a method for creating incisions in the muscles of the bladder neck by infarction, operative in accordance with a further embodiment of the disclosed technique. In procedure 300, the location of the bladder neck of a patient is found. With reference to Figure 3A, the physician inserts overtube 164 into the urethra of the patient until balloon 158 is inside bladder 152. The physician inflates balloon 158 and pulls overtube 164 back until balloon 158 is blocked by the bladder neck.

In procedure 302, a radial cutter implant is delivered to the constricted location. With reference to Figure 3B and 5A, the physician inserts delivery 176 into positioning tube 162 and delivers radial cutter implant 240 to the location of the bladder neck (i.e., the constricted location). It is noted that implant 240 is located such that it does not extend into the bladder itself, thereby avoiding bladder irritation. Alternatively, the physician delivers the implant to a different constricted location within the urinal system, and outside of the bladder, for relieving the constriction. In procedure 304, the radial cutter implant is released and the delivery system is removed. The implant is released where it does not extend distally beyond the bladder neck and into the bladder itself, thereby avoiding bladder irritation. With reference to Figure 3C and 5A, the physician exposes radial cutter implant 240 from implant sheath 166. Radial cutter implant 240 expands and attaches itself to the surrounding tissues (i.e., the closed shape wires of radial cutter implant 240 are attached to the tissues surrounding the implant and apply pressure thereon). The physician removes delivery system 150 from urethra 154 of the patient.

In procedure 306, continuous pressure is applied on the tissues surrounding the implant by employing the radial cutter implant, and without irritating the bladder. With reference to Figure 3C and 5A, the closed shape wires of radial cutter implant 240 apply continuous pressure on the surrounding tissues. Implant 240 does not irritate the bladder since it does not come into contact there-with.

In procedure 308, at the appearance of a predetermined condition, the radial cutter implant is extracted from the patient. With reference to Figure 3C and 5A, the physician extracts radial cutter implant 240 from the patient at the appearance of a predetermined condition. The predetermined condition can be the passage of a predetermined period of time, the appearance of desired incisions on the surrounding tissues, the appearance of a predetermined physiological effect, and the like.

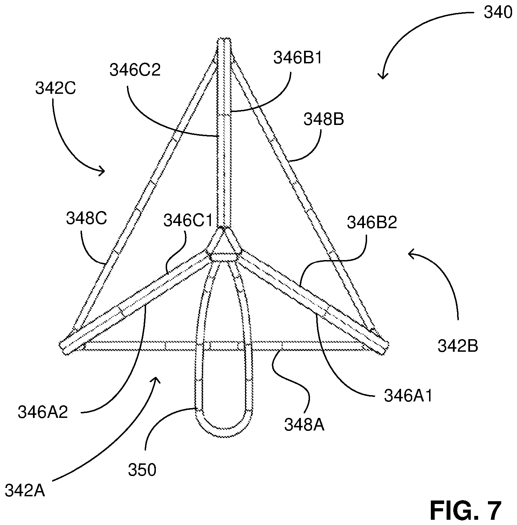

Reference is now made to Figure 7, which is a schematic illustration of a radial cutter implant, generally referenced 340, depicted from a top view perspective, constructed and operative in accordance with a further embodiment of the disclosed technique. Implant 340 is constructed from three closed shaped wires 342A, 342B and 342C (also referred to herein below, together, as wires 342), and further includes an anchoring leaflet 350. Each of wires 342 is adjacently attached between the other ones of wires 342, as would be detailed further below. Anchoring leaflet 350 extends from a proximal end (not referenced) of implant 340, and is positioned between two of wires 342.

Each of wires 342A, 342B and 342C includes three sections: a narrow U-shaped proximal end; two radially extending straight arms - extending in the radial-distal from the distal ends (not referenced) of the U-shaped proximal end; and a support crosspiece connecting the distal ends of the radially extending straight arms. In particular, closed shape wire 342A includes a U-shaped proximal end 344A, two radially extending straight arms 346A1 and 346A2, and a support crosspiece 348A.

Radially extending straight arms 346A1 and 346A2 extend in the radial-distal direction from the distal ends (not referenced) of U-shaped proximal end 344A. Support crosspiece 348A is coupled between the distal ends (not referenced) of radially extending straight arms 346A1 and 346A2. The respective sections of wires 342B and 342C are similarly constructed denoted.

Wires 342 form together a triangular pyramid with the U-shaped proximal heads of the wires forming the apex of the pyramid and the support crosspieces of the wires forming the base of the pyramid. Specifically, support crosspieces 348A, 348B, and 348C form together a support frame (not referenced) defining the base of pyramid-shaped implant 340. The pyramidal shape of implant 340, and in particular the support frame formed by the support crosspieces, provides structural solidity to implant 340. Specifically, the support frame provides resistance to forces applied on implant 340 by the surrounding tissues of the bladder neck, when implanted within the bladder neck. Thus, the support frame maintains the structural stability of the implant, and enables the radially extending straight arms to continue to apply the incising force on the surrounding tissues.

Implant 340 is inserted and extracted in a similar manner, to that described above with reference to Figures 1, 2, 3A-C, 4A-C and 5A-C. Radial cutter implant 340 may be temporary in the sense that it may be removed from the urethral at a selected time after deployment. Additionally, implant 340 is made from similar materials to the implants describes above.

In accordance with an alternative embodiment of the disclosed technique, implant 340 can comprise other numbers of closed shaped wires, and at least one closed shape wire, such as four wires, five wires, and the like. If there is more than a single wire, each wire is adjacently attached between two adjacent wires as described above. Similarly, for any number of closed shape wires, the support crosspieces thereof form together a support frame providing structural solidity to implant, as described above.

Radial cutter implant 340 is located and expanded such that it does not extend distally beyond the bladder neck of the patient (i.e., does not extend into the bladder). Specifically, the support frame formed of the support crosspieces is located within the bladder neck before the bladder, and does not come into contact with the tissues of the bladder itself. Thereby, implant 380 does not irritate the bladder of the patient.

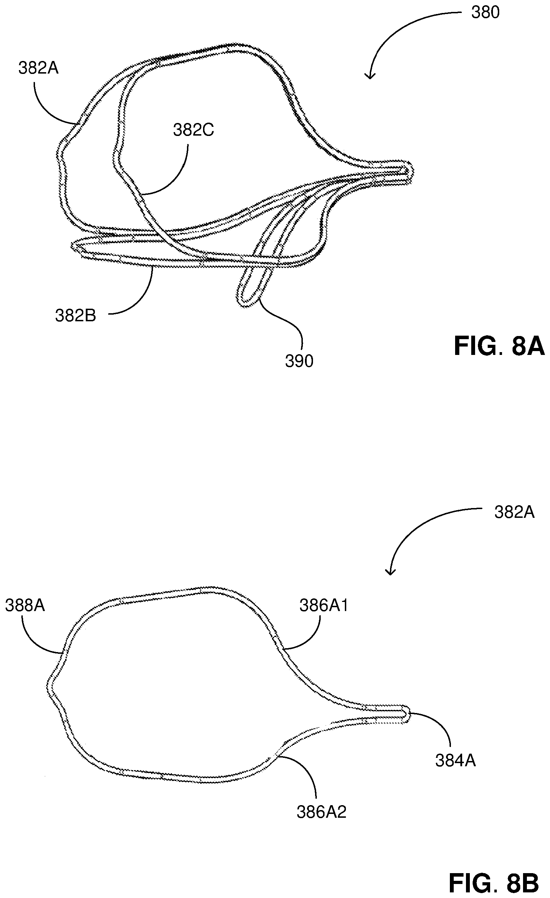

Reference is now made to Figures 8A and 8B, which are schematic illustrations of a radial cutter implant, generally referenced 380, constructed and operative in accordance with another embodiment of the disclosed technique. Figure 8A depicts implant 380 from an isometric perspective, and Figure 8B depicts a closed shape wire of the implant of figure 8A from a side view perspective.

Implant 380 is composed of three closed shaped wires 382A, 382B and 382C (also referred to herein below, together, as wires 382), and further includes an anchoring leaflet 390. Each of wires 382 is adjacently attached between the other ones of wires 382, as would be detailed further below. Anchoring leaflet 390 extends from a proximal end (not referenced) of implant 380, and is positioned between two of wires 382.

Each of wires 382A, 382B and 382C includes three sections: a narrow U-shaped proximal end; two radially extending arms - extending radially and distally from the distal ends (not referenced) of the U-shaped proximal end; and a support crosspiece connecting the distal ends of the radially extending arms. In particular and as seen in Figure 8B, closed shape wire 382A includes a U-shaped proximal end 384A, two radially extending arms 386A1 and 386A2, and a support crosspiece 388A.

Radially extending arms 386A1 and 386A2 extend radially and distally from the distal ends (not referenced) of U-shaped proximal end 384A. Support crosspiece 388A is coupled between the distal ends (not referenced) of radially extending arms 386A1 and 386A2. The components of wires 382B and 382C are similarly constructed and denoted. Support crosspieces 388A, 388B, and 388C form together a support frame (not referenced). The support frame provides structural solidity to implant 380, and enables the radially extending arms to continue to apply the incising force on the surrounding tissues.

In accordance with an alternative embodiment of the disclosed technique, implant 380 can comprise other numbers of closed shaped wires, and at least one closed shape wire, such as four wires, five wires, and the like. If there is more than a single wire, each wire is adjacently attached between two adjacent wires as described above. Similarly, for any number of closed shape wires, the support crosspieces thereof form together a support frame providing structural solidity to implant, as described above.

Radial cutter implant 380 is located and expanded such that it does not extend distally beyond the bladder neck of the patient (i.e., does not extend into the bladder). Specifically, the support frame formed of the support crosspieces is located within the bladder neck before the bladder, and does not come into contact with the tissues of the bladder itself. Thereby, implant 380 does not irritate the bladder of the patient.

Implant 380 is inserted and extracted in a similar manner, to that described above with reference to Figures 1, 2, 3A-C, 4A-C and 5A-C. Additionally, implant 380 is made from similar materials to the implants describes above. Radial cutter implant 380 may be temporary in the sense that it may be removed from the urethral at a selected time after deployment.

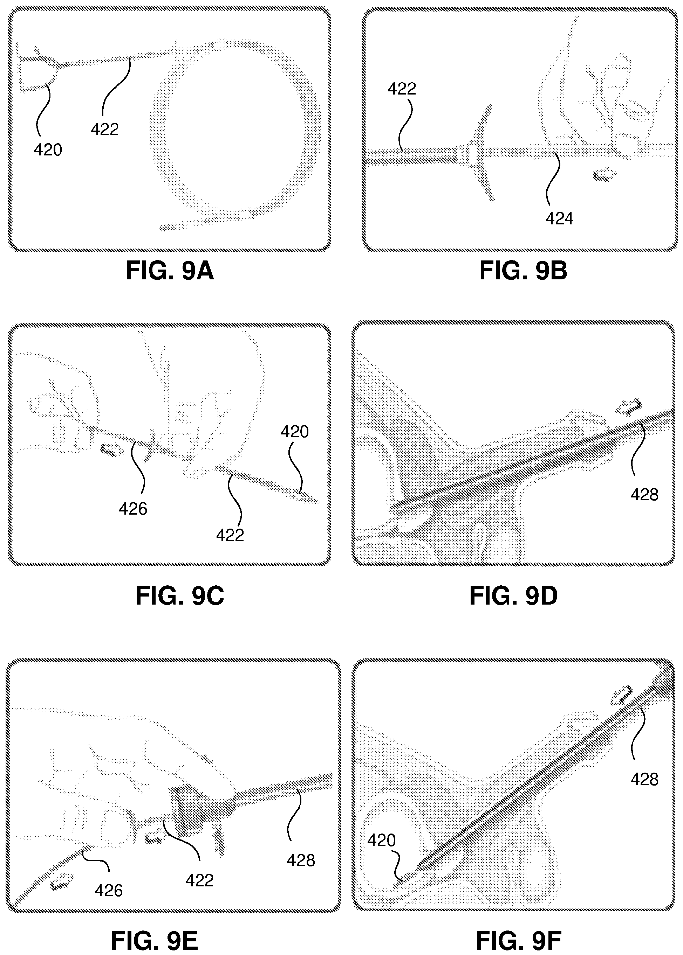

Reference is now made to Figures 9A-9L, which are schematic illustrations of a step-by-step method for delivering, and for extracting, a radial cutter implant, generally referenced 420, operative in accordance with a further embodiment of the disclosed technique. With reference to Figure 9A, the implant 420 is delivered when enfolded in an implant sheath 422, as detailed below. With reference to Figure 9B, the physician removes a protective cover 424 from implant 420. Protective cover 424 keeps implant 420 sterile during storage prior to use.

With reference to Figure 9C, while holding a guidewire 426, the physician pushes implant sheath 422 over implant 420 thereby enfolding implant 420 within sheath 422 for delivery into the urethra. With reference to Figure 9D, the physician inserts a rigid cystoscope 428 (e.g., size 20French) into the urethra, for example, as in a routine urethral catheterization procedure. With reference to Figure 9E, the physician inserts the folded implant 420 into cystoscope 428. Using guidewire 426, the physician continues pushing implant 420 through cystoscope 428 until implant 420 reaches the distal end of cystoscope 428 and opens up in the bladder.

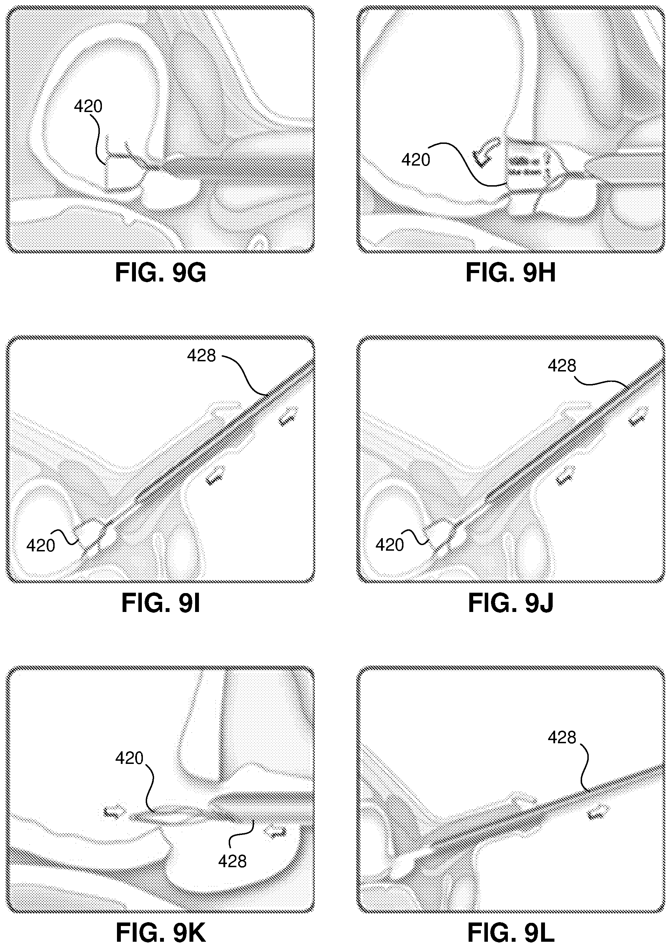

With reference to Figure 9F, the physician removes sheath 422 from implant 420 and out of cystoscope 428. With reference to Figure 9G, implant 420 expands (or is being expanded). With reference to Figure 9H, the physician rotates guidewire 426 (Figure 9E) and brings implant 420 to the correct orientation. The closed shape wires of implant 420 can be color coded, such that the sections that should be positioned on the top are colored, for example, blue; and the sections that should be positioned on the bottom are colored, for example, white. With reference to Figure 9I, While holding implant 420 in place, the physician retracts cystoscope 428. Thereby, implant 420 is implanted within the bladder neck and starts applying radial outward force on the surrounding tissues by the closed shape wires for inducing incisions within the surrounding tissues. Implant 420 is left within the bladder neck for a selected time period (e.g., ranging between one hour and one month). Thereafter implant 420 is removed as would be detailed below. Alternatively, implant 420 is made of biodegradable materials and simply dissolves after a selected time period.

When implanted within the bladder neck and the urethra of the subject, implant 420 applies radial outward force on the tissues surrounding it, for producing incisions in the surrounding tissues. Thereby, implant 420 relieves a prostate enlargement problem by cutting through the tissues and extending the urinal passage (i.e., the wires both incise and extend the tissues in the radial direction from the urethra axis outwardly).

With reference to Figure 9J, the physician inserts cystoscope 428 through the urethra toward implant 420. Cystoscope 428 contains therein a coupler (not shown) designed to hook onto the proximal end of implant 420. With reference to Figure 9K, the physician employs the coupler for hooking onto implant 420 and for pulling implant 420 into cystoscope 428, thereby enfolding implant 420 within cystoscope 428. Alternatively, an implant sheath can be employed instead of cystoscope 428. With reference to Figure 9L, once implant 420 is fully enfolded within cystoscope 428, the physician pulls cystoscope 428 out of the urethra of the subject.

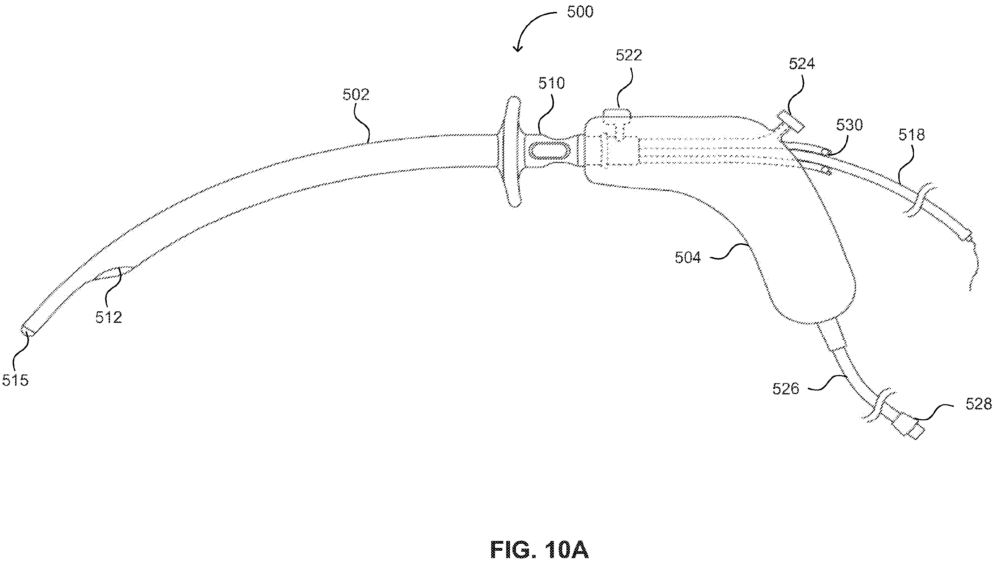

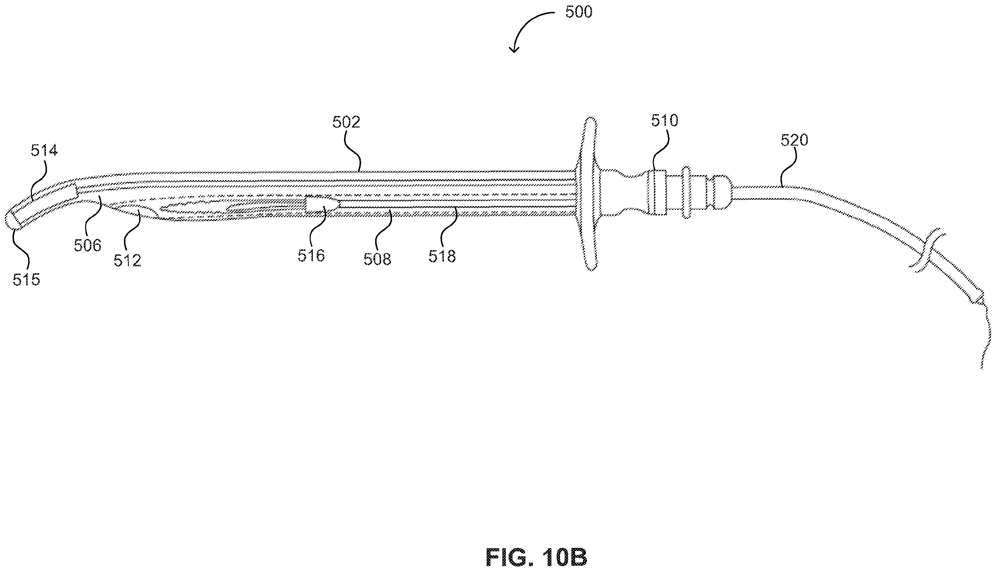

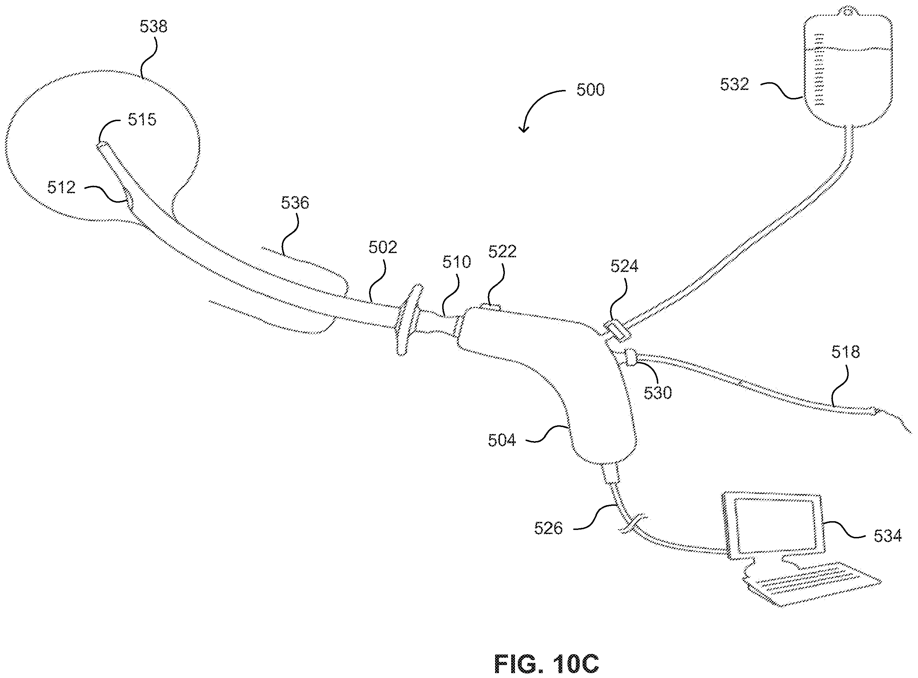

Reference is now made to Figures 10A, 10B and 10C, which are schematic illustrations of a system, generally referenced 500, for delivering a urethral implant to the bladder neck of a patient, constructed and operative in accordance with another embodiment of the disclosed technique. Delivery system 500 includes an overtube 502, and a handle 504. Overtube 502 includes two elongated passages, a first elongated passage 506 and a second elongated passage 508 and a handle connector 510. Second elongated passage 508 includes an exit port 512. Delivery system 510 further includes a camera 514 and a urethral implant 516. Handle connector 510 is configured to connect with a handle 502.

Camera 514 is located at the distal end 515 of first elongated passage 506, and the wiring of camera 514 is located within first elongated passage 506. The optical axis of camera 514 may be aligned with axis of overtube 502. Alternatively, the optical axis of camera 514 may be at an angle (e.g., 10 degrees, 30 degrees, 45 degrees and the like) relative to the axis overtube 502. Urethral implant 516 is pre-loaded (i.e., prior to the procedure) within the distal end of second elongated passage 508 of overtube 502. Urethral implant 516 is coupled with a guidewire 518, located within second elongated passage 508. Guidewire 518 extends toward the proximal end of overtube 508 and exits handle connector 510 at the proximal end of overtube 502. Second elongated passage 508 may further be employed to deliver fluids to or from the urethra or the bladder.

Handle 504 includes a connector lock 522, a saline delivery port 524, an optics cable 526, an optics connector 528, and a guidewire exit port 530.

Prior to delivery of urethral implant 516, the subject may receive medication to relieve anxiety. The subject may also receive prophylactic antibiotics per local hospital practice. Also prior to deploying urethral implant 516, a physician connects handle 504 to saline 532 via saline port 524, and to a display monitor 534 via optics connector 528. Then the physician connects handle connector 510 of overtube 502 to handle 504 by first inserting urethral implant 516 into the distal end of handle 504 until guidewire 518 exits guidewire exit port 530, and overtube handle connector 510 locks with connector lock 522.

As depicted in Figure 10C, the physician inserts overtube 502 into the penis 536 of the patient and through the urethra of patient, until exit port 512 is positioned within the bladder 538 of the patient. The physician fills the bladder of the patient with saline and then pushes guidewire 518 until urethral implant 516 exits overtube 502 through exit port 512, and enters bladder 538 of the patient. The physician withdraws handle 504 until camera 514 reaches the bladder neck of the patient and urethral implant 516 comes into view. The physician maneuvers urethral implant 516 by turning guidewire 518. The physician then pulls guidewire 518 to position urethral implant 516 into the patient's prostatic urethra. The physician than pulls overtube 502 out of penis 536 while urethral implant 516 stays in the prostatic urethral with a wire extending from the penis to enable removal of the implant at a later time. Urethral implant 516 may be temporary in the sense that it may be removed from the urethral at a selected time after deployment. Also, system 500 is disposable. In other words, once the urethral implant 516 is deployed and overtube 502 is retracted from penis 536, overtube 502 may be disposed along with camera 514. Also, urethral implant 516 may be a temporary radial cutter such as described above.

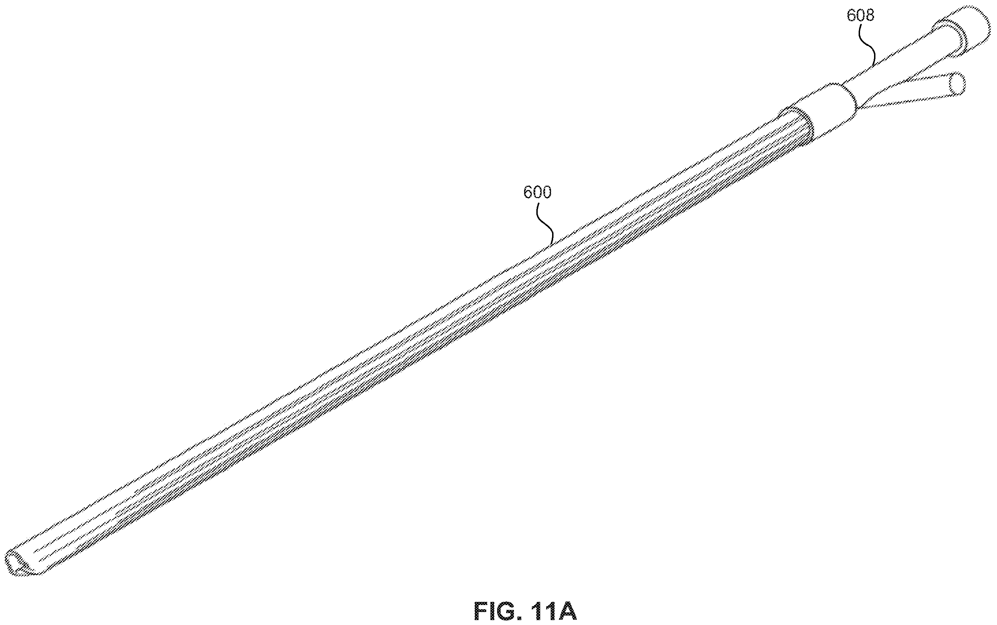

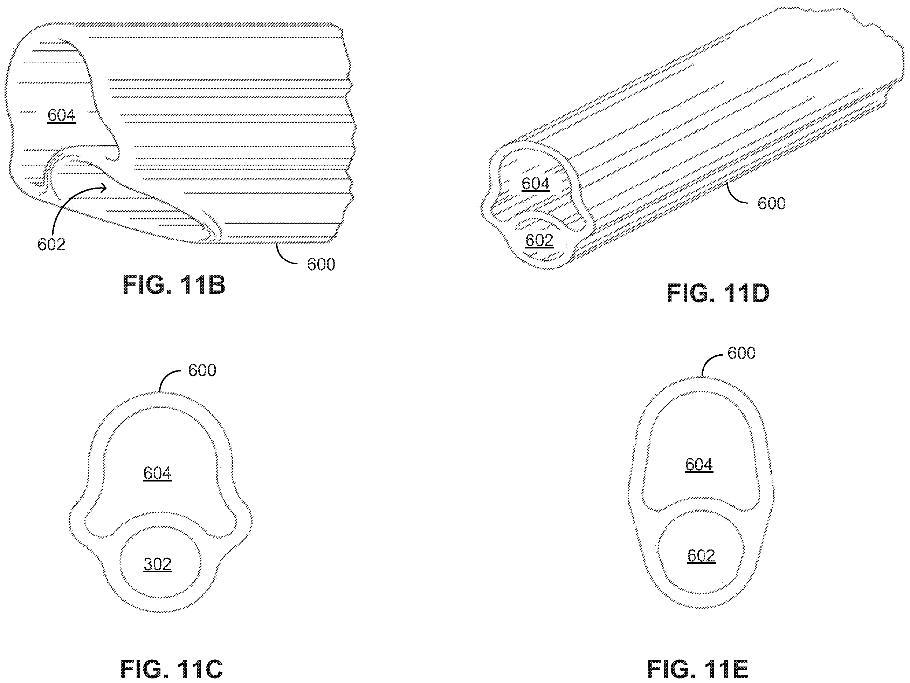

Reference is now made to Figures 11A-11E, which are a schematic illustrations of an overtube, generally referenced 600, according to a further embodiment of the disclosed technique. Overtube 600 may be employed in a delivery system such as described above in conjunction with Figure 10A-10C. Overtube 600 includes a first elongated passage 602, a second elongated passage 602 and a handle connector 606. Figures 11A depicts an isometric view overtube 600. Figure 11B, depicts an isometric view of the distal end of over tube 600. Figures 11C depicts a front view of the distal end of overtube 600. Figures 11D depicts an isometric view of the proximal end of overtube 600 and Figures 11E depicts a back view of the proximal end of overtube 600. In overtube 600 first elongated passage 602 and second elongated passage 604 are separate creating two lumens.

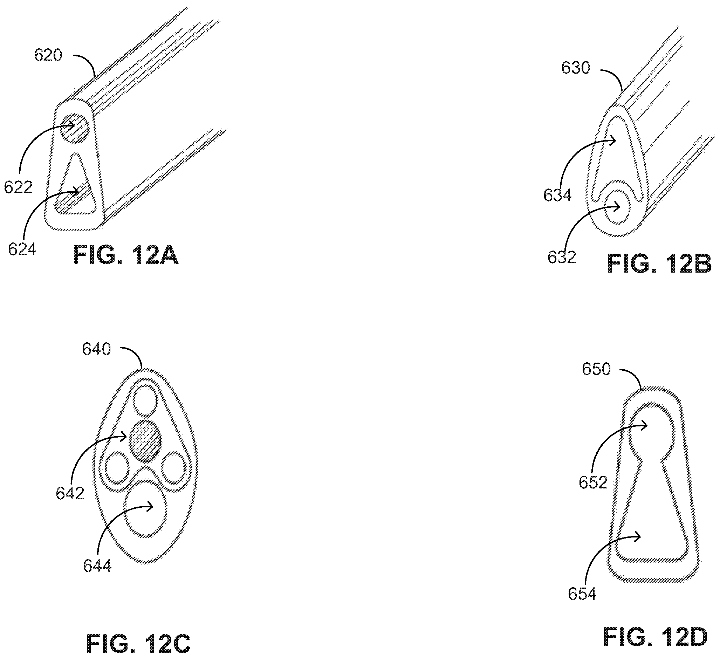

In general, the first elongated passage and the second elongated passage may exhibit different configurations. Reference is now made to Figures 12A-12D, which are schematic illustrations of different cross section configurations of an overtube, in accordance with another embodiment of the disclosed technique. With reference to Figure 12A, overtube 620 includes first elongated passage 622 and second elongated passage 624. With reference to Figure 12B, overtube 630 includes first elongated passage 632 and second elongated passage 634. With reference to Figure 12C, overtube 640 includes first elongated passage 642 and second elongated passage 644. In Figures 12A-12C, the first and second elongated passages are separate creating two lumens. With reference to Figure 12D, overtube 650 includes first elongated passage 652 and second elongated passage 654. In Figure 12D, first elongated passage 652 and second elongated passage 654 are connected creating a single lumen.

It will be appreciated by persons skilled in the art that the disclosed technique is not limited to what has been particularly shown and described hereinabove. Rather the scope of the invention is defined only by the claims, which follow.

## Claims

1. A urethral implant delivery system (500) comprising:
a handle (504);
an overtube (502), including a first elongated passage (506), a second elongated passage (508), and a handle connector (510);
a camera (514), located at a distal end (515) of said first elongated passage;
a urethral implant (516), located within said second elongated passage; and
a guidewire (518), coupled with said urethral implant, said guidewire enables to push said urethral implant through said overtube until said urethral implant exits said overtube, said guidewire extends toward a proximal direction of said overtube and exits said handle;
wherein said handle connector is configured to be coupled with said handle (504).

2. The urethral implant delivery system according to claim 1, wherein said urethral implant includes at least one wire having a closed-shape, said urethral implant exhibiting an expanded configuration and being foldable into a folded configuration, said urethral implant is configured to expand from said folded configuration to said expanded configuration, wherein said urethral implant is configured to be implanted within a restricted location of a urethra (154), and for applying pressure on surrounding tissue of said urethra.

3. The urethral implant delivery system according to claim 2, wherein said urethral implant is configured for creating incisions in said urethra.

4. The urethral implant delivery system according to claim 1, wherein said urethral implant is configured for implantation in a prostatic urethra.

5. The urethral implant delivery system according to claim 2, wherein said urethral implant further includes an anchoring leaflet (250; 350; 390) configured to prevent said urethra implant from moving in a direction of extension of said anchoring leaflet.

6. The urethral implant delivery system according to claim 1, wherein said urethral implant is removable from an implantee.

7. The urethral implant delivery system according to claim 1, wherein said urethral implant delivery system is for single use.

8. The urethral implant delivery system according to claim 1, wherein said urethral implant is configured for temporary implantation.

9. The urethral implant delivery system according to claim 1, wherein said second elongated passage is further configured to be employed to transport fluids to or from a urethra or a bladder (152; 538).

10. The urethral implant delivery system according to claim 1, wherein said handle includes a connector lock (522), a saline delivery port (524), an optics cable (526), an optics connector (528), and a guidewire exit port (530).

11. The urethral implant delivery system according to claim 1, wherein an optical axis of said camera is aligned with an axis said overtube.

12. The urethral implant delivery system according to claim 1, wherein an optical axis of said camera is at an angle shift relative to an axis of said overtube.

13. The urethral implant delivery system according to claim 1, wherein said guidewire is rotatable for maneuvering said urethral implant at a desired orientation.

14. The urethral implant delivery system according to claim 1, wherein said guidewire enables to position said urethral implant into a prostatic urethra by pulling said guidewire.

## Patentansprüche

1. System zur Einführung eines Harnröhrenimplantats (500), umfassend:
einen Griff (504);
ein Überrohr (502), das einen ersten länglichen Durchgang (506), einen zweiten länglichen Durchgang (508) und einen Griffverbinder (510) aufweist;
eine Kamera (514), die sich an einem distalen Ende (515) des ersten länglichen Durchgangs befindet;
ein Harnröhrenimplantat (516), das sich in dem zweiten länglichen Durchgang befindet; und
einen Führungsdraht (518), der mit dem Harnröhrenimplantat gekoppelt ist, wobei der Führungsdraht ermöglicht, das Harnröhrenimplantat durch das Überrohr voranzutreiben, bis das Harnröhrenimplantat das Überrohr verlässt, wobei sich der Führungsdraht in Richtung einer proximalen Richtung des Überrohrs erstreckt und den Griff verlässt;
wobei der Griffverbinder dazu ausgelegt ist, mit dem Griff (504) gekoppelt zu werden.

2. System zur Einführung eines Harnröhrenimplantats nach Anspruch 1, wobei das Harnröhrenimplantat mindestens einen Draht mit einer geschlossenen Form aufweist, wobei das Harnröhrenimplantat eine ausgedehnte Auslegung zeigt und in eine gefaltete Auslegung faltbar ist, wobei das Harnröhrenimplantat dazu ausgelegt ist, sich aus der gefalteten Auslegung in die ausgedehnte Auslegung auszudehnen, wobei das Harnröhrenimplantat dazu ausgelegt ist, in eine eingeschränkte Stelle einer Harnröhre (154) implantiert zu werden, und um Druck auf umgebendes Gewebe der Harnröhre aufzubringen.

3. System zur Einführung eines Harnröhrenimplantats nach Anspruch 2, wobei das Harnröhrenimplantat zum Erzeugen von Inzisionen in der Harnröhre ausgelegt ist.

4. System zur Einführung eines Harnröhrenimplantats nach Anspruch 1, wobei das Harnröhrenimplantat zur Implantation in eine prostatische Harnröhre ausgelegt ist.

5. System zur Einführung eines Harnröhrenimplantats nach Anspruch 2, wobei das Harnröhrenimplantat ferner ein Verankerungssegel (250; 350; 390) aufweist, das dazu ausgelegt ist, zu verhindern, dass sich das Harnröhrenimplantat in eine Erstreckungsrichtung des Verankerungssegels bewegt.

6. System zur Einführung eines Harnröhrenimplantats nach Anspruch 1, wobei das Harnröhrenimplantat entfernbar ist.

7. System zur Einführung eines Harnröhrenimplantats nach Anspruch 1, wobei das System zur Einführung eines Harnröhrenimplantats für den einmaligen Gebrauch ist.

8. System zur Einführung eines Harnröhrenimplantats nach Anspruch 1, wobei das Harnröhrenimplantat zur vorübergehenden Implantation ausgelegt ist.

9. System zur Einführung eines Harnröhrenimplantats nach Anspruch 1, wobei der zweite längliche Durchgang ferner dazu ausgelegt ist, verwendet zu werden, um Fluids zu oder von einer Harnröhre oder einer Blase (152; 538) zu transportieren.

10. System zur Einführung eines Harnröhrenimplantats nach Anspruch 1, wobei der Griff eine Verbinderverriegelung (522), eine Öffnung zur Zufuhr von Kochsalz (524), ein optisches Kabel (526), einen optischen Verbinder (528) und eine Führungsdrahtaustrittsöffnung (530) aufweist.

11. System zur Einführung eines Harnröhrenimplantats nach Anspruch 1, wobei eine optische Achse der Kamera auf eine Achse des Überrohrs ausgerichtet ist.

12. System zur Einführung eines Harnröhrenimplantats nach Anspruch 1, wobei eine optische Achse der Kamera in Winkelverschiebung bezogen auf eine Achse des Überrohrs ist.

13. System zur Einführung eines Harnröhrenimplantats nach Anspruch 1, wobei der Führungsdraht zum Manövrieren des Harnröhrenimplantats in einer gewünschten Ausrichtung drehbar ist.

14. System zur Einführung eines Harnröhrenimplantats nach Anspruch 1, wobei der Führungsdraht ermöglicht, das Harnröhrenimplantat durch Ziehen des Führungsdrahts in eine prostatische Harnröhre zu positionieren.

## Revendications

1. Système de pose d'implant urétral (500) comprenant :
une poignée (504) ;
un surtube (502), comprenant un premier passage allongé (506), un second passage allongé (508), et un connecteur de poignée (510) ;
une caméra (514), située à une extrémité distale (515) dudit premier passage allongé ;
un implant urétral (516), situé à l'intérieur dudit second passage allongé ; et
un fil-guide (518), couplé audit implant urétral, ledit fil-guide permettant de pousser ledit implant urétral à travers ledit surtube jusqu'à ce que ledit implant urétral sorte dudit surtube, ledit fil-guide s'étendant vers une direction proximale dudit surtube et de ladite poignée ;
ledit connecteur de poignée étant configuré pour être couplé à ladite poignée (504).

2. Système de pose d'implant urétral selon la revendication 1, ledit implant urétral comprenant au moins un fil ayant une forme fermée, ledit implant urétral présentant une configuration étendue et pouvant être plié dans une configuration pliée, ledit implant urétral étant configuré pour s'étendre de ladite configuration pliée à ladite configuration étendue, ledit implant urétral étant configuré pour être implanté à l'intérieur d'un emplacement restreint d'un urètre (154), et pour appliquer une pression sur le tissu environnant dudit urètre.

3. Système de pose d'implant urétral selon la revendication 2, ledit implant urétral étant configuré pour créer des incisions dans ledit urètre.

4. Système de pose d'implant urétral selon la revendication 1, ledit implant urétral étant configuré pour être implanté dans un urètre prostatique.

5. Système de pose d'implant urétral selon la revendication 2, ledit implant urétral comprenant en outre un feuillet d'ancrage (250 ; 350 ; 390) configuré pour empêcher ledit implant urétral de se déplacer dans une direction d'extension dudit feuillet d'ancrage.

6. Système de pose d'implant urétral selon la revendication 1, ledit implant urétral étant amovible d'un emplacement d'implantation.

7. Système de pose d'implant urétral selon la revendication 1, ledit système de pose d'implant urétral étant à usage unique.

8. Système de pose d'implant urétral selon la revendication 1, ledit implant urétral étant configuré pour une implantation temporaire.

9. Système de pose d'implant urétral selon la revendication 1, ledit second passage allongé étant en outre configuré pour être employé pour transporter des fluides vers ou depuis un urètre ou une vessie (152 ; 538).

10. Système de pose d'implant urétral selon la revendication 1, ladite poignée comprenant un verrou de connecteur (522), un port d'administration de solution saline (524), un câble optique (526), un connecteur optique (528) et un port de sortie de fil-guide (530).

11. Système de pose d'implant urétral selon la revendication 1, un axe optique de ladite caméra étant aligné avec un axe dudit surtube.

12. Système de pose d'implant urétral selon la revendication 1, un axe optique de ladite caméra étant décalé d'un angle par rapport à un axe dudit surtube.

13. Système de pose d'implant urétral selon la revendication 1, ledit fil-guide étant rotatif pour manœuvrer ledit implant urétral selon une orientation souhaitée.

14. Système de pose d'implant urétral selon la revendication 1, ledit fil-guide permettant de positionner ledit implant urétral dans un urètre prostatique en tirant ledit fil-guide.
